(19) Europäisches Patentamt
European Patent Office
Office européen des brevets

(11) Veröffentlichungsnummer: **0 100 458 B1**

# (12) EUROPÄISCHE PATENTSCHRIFT

(45) Veröffentlichungstag der Patentschrift: **06.06.90**

(51) Int. Cl.[5]: **A 61 L 15/16,** A 61 K 31/785, A 61 K 31/74

(21) Anmeldenummer: **83106647.7**

(22) Anmeldetag: **07.07.83**

## (54) Mittel zur Behandlung von Wunden.

(30) Priorität: **14.07.82 DE 3226753**

(43) Veröffentlichungstag der Anmeldung:
**15.02.84 Patentblatt 84/07**

(45) Bekanntmachung des Hinweises auf die Patenterteilung:
**06.06.90 Patentblatt 90/23**

(84) Benannte Vertragsstaaten:
**AT BE CH DE FR GB IT LI LU NL SE**

(56) Entgegenhaltungen:
**EP-A-0 026 988**
**EP-A-0 039 542**
**EP-A-0 074 179**
**US-A-4 226 232**
**US-A-4 272 518**

(73) Patentinhaber: **SMITH & NEPHEW MEDICAL LIMITED**
**101 Hessle Road**
**Hull (GB)**

(72) Erfinder: **Wündisch, Karl**
**c/o RA Krüger Mommsenstrasse 65**
**D-1000 Berlin 12 (DE)**
Erfinder: **Zimmermann, Ingfried, Dr.**
**Gollanczstrasse 28 c**
**D-1000 Berlin 28 (DE)**
Erfinder: **Dobinsky, Helmar, Dr.**
**Dachsberg 17 b**
**D-1000 Berlin 33 (DE)**
Erfinder: **Günzel, Peter, Dr.**
**Hubertusstrasse 19**
**D-1000 Berlin 20 (DE)**
Erfinder: **Schoebel, Christel, Dr.**
**Möllerpfad 11**
**D-1000 Berlin 37 (DE)**

(74) Vertreter: **Koepsell, Helmut, Dipl.-Ing.**
**Mittelstrasse 7**
**D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 100 458 B1

**Beschreibung**

Mittel zur Behandlung von Entzündungen von Wunden sind vorbekannt. So findet neben den konventionellen textilen Verbänden unter anderem ein Produkt auf Dextran-Basis Anwendung bei der Behandlung von Wunden (US-Patent 4,225,580). Dieses Material hat aber den Nachteil, daß es relativ schwierig zu applizieren ist, und insbesondere den Nachteil, daß es relativ schwierig aus der Wunde zu entfernen ist, da die kleinen Dextrankörner leicht mit den Wundrändern verkleben.

Die EP-A-0 039 542 beschreibt Salze aluminiumhydrolysierter Stärke-Polyacrylnitril-propfcopolymerisate ohne Zusatz von Polyalkylenglykolen zur Herstellung von beispielsweise Bandagen. Aus der US-A-4 272 518 ist eine Paste zur Wundbehandlung mittels eines hydrophilen, wasserquellbaren Polymeren und Polyalkylenglykolen bekannt. Die US-A-4 226 232 offenbart einen wasseraufnehmenden Wundverband aus einem hydrolysierten Stärke-Polyacrylnitril-propfcopolymerisat mit freien oder durch Alkalimetalle abgesättigten Carboxylgruppen.

Es wurde nun gefunden, daß Mittel zur Wundbehandlung, die 1 bis 4 Gewichtsprozent eines Pfropf-Copolymerisats aus Stärke und hydrolysiertem Polyacrylnitril, dessen Carboxygruppen-Wasserstoffe zu 5 bis 90% durch Aluminium substituiert sind, in einer 2 bis 50 gewichtsprozentigen wässrigen Di- oder Polyhydroxyalkan-Lösung (jeweils bezogen auf das Gesamtgewicht des Mittels) enthalten, wesentlich günstigere Eigenschaften im Vergleich zu den zur gleichen Anwendung bestimmten vorbekannten Mitteln haben.

Das für das erfindungsgemäße Mittel verwendete Pfropf-Copolymerisat aus Stärke und hydrolisiertem Polyacrylnitril, dessen Carboxygruppen-Wasserstoffe zu 5 bis 90% durch Aluminium substituiert sind, kann beispielsweise nach der Lehre des US-Patentes 4,302,369 hergestellt werden, indem man unter den dort angegebenen Bedingungen eine Stärkesuspension mit Acetonitril in Gegenwart eines chemischen Initiators wie Cerammoniumnitrat umsetzt, das entstandene Pfropf-Copolymere mit starken Basen hydrolysiert und dann mit Aluminiumsalzen umsetzt. Das erhaltene Produkt kann dann getrocknet, mit alkoholischer Ammoniaklösung gewaschen und mit Salzsäure auf den gewünschten pH-Wert von 6,0 bis 7,5 eingestellt werden. Andererseits ist es aber auch möglich, dieses Produkt beispielsweise von der Henkel Corporation, Minneapolis z.B. unter der Bezeichnung SGP 157 M käuflich zu erwerben.

Zur Herstellung des erfindungsgemäßen Mittels sind solche Pfropf-Copolymerisate geeignet, die folgende Merkmale aufweisen.

Das Verhältnis der Stärkekomponente zur Summe von Acrylat- und Acrylamidkomponente beträgt im Copolymerisat vorzugsweise 1:3 bis 1:0,9 Gewichtsprozent.

Das Verhältnis von Carboxylgruppen zu Amidgruppen ist vorzugsweise 2:1 bis 9:1 mol:mol.

Im Copolymerisat sind vorzugsweise 25 bis 75% der Carboxylgruppen durch Aluminium substituiert.

Die Korngröße des Copolymerisats ist vorzugsweise 0,01 bis 0,5 mm.

Dihydroxyalkane mit 2 bis 6 Kohlenstoffatomen, die sich zur Herstellung des erfindungsgemäßen Mittels eigenen, sind beispielsweise 1,2-Dihydroxyethan, 1,2-Dihydroxypropan, 2,3-Dihydroxybutan und 3,4-Dihydroxyhexan.

Zur Herstellung des erfindungsgemäßen Mittels geeignete Polyhydroxyalkane der allgemeinen Formel

$$C_nH_{(2n+2)}O_n$$

worin n die Ziffern 3 bis 6 bedeuten, sind beispielsweise Glycerin, Sorbit, Mannit, Adonit, Ribit, Dulcit, Erythrit und Xylit.

Polyethylenglykol, das zur Herstellung des erfindungsgemäßen Mittels geeeignet ist, ist ein solches mit einem Molekulargewicht von 200 bis 600. Das für das Mittel verwendbare Polypropylenglykol hat vorzugsweise ein Molgewicht von 200 bis 450.

Das Di- oder Polyhydroxyalkan wird im erfindungsgemäßen Mittel zu maximal 50 Gewichtsprozent und vorzugsweise in einer Konzentration von 10 bis 30 Gewichtsprozent, angewendet. Die Verdünnung der Diole oder Polyole erfolgt vorzugsweise mittels demineralisiertem Wasser.

Zur Herstellung der Mittel werden die Komponenten beispielsweise mittels eines Rührers gleichmäßig vermischt und mittels Hitze sterilisiert.

Das erfindungsgemäße Mittel zur Behandlung von Wunden ist bezüglich seiner Zweckbestimmung nicht nur zur Behandlung von Wunden im engeren Sinne geeignet, sondern wie die in der vom Bundesverband der pharmazeutischen Industrie e.V., D-6000 Frankfurt/Main, herausgegebenen "Rote Liste - 1980", aufgeführten Wundbehandlungsmittel zur Behandlung zahlreicher mit einer Sekretausscheidung verbundenen Entzündungen oder Verletzungen der Haut oder Schleimhaut geeignet. Solche Erkrankungen oder Verletzungen sind beispielsweise Schnittwunden, Stoßwunden, Rißwunden, Quetschwunden. Brandwunden, Frostwunden, Abschürfungen, Sonnenbrand, Ekzeme, Hämorrhoiden, etc.

Das erfindungsgemäße Mittel zur Behandlung von Wunden kann zusätzlich noch die bei solchen Mitteln üblichen Zusatzstoffe und Hilfsstoffe (z.B. Duftstoffe) sowie darüberhinaus noch Wirkstoffe, wie sie in diesen Mitteln üblicherweise eingesetzt werden, enthalten. Derartige Wirkstoffe sind z.B. Bakteriostatika (wie zum Beispiel Sulfonamide, wie Sulfadiazin, Sulfatolamid oder Antibiotika wie Penicilin, Neomycin oder Chloramphenicol), Antimykotika wie z.B. Salicylsäure und deren Derivate wie Silicylhydroxamsäure und Salicylamid oder Miconacol und Isoconacol und Lokalanesthetika wie z.B. Ester der p-Aminobenzoesäure, wie der Methylester oder Ethylester oder Lidocain etc.

Das Mittel wird auf die zu behandelnde Stelle

des Körpers aufgetragen, und gegebenenfalls mit Gaze abgedeckt.

Das erfindungsgemäße Mittel zeichnet sich durch einen außergewöhnlichen Saugdruck aus und begünstigt demzufolge, daß die Wunde infolge Wasserentzugs rasch heilt.

Durch die im erfindungsgemäßen Mittel enthaltenen Di- oder Polyhydroxyalkane wird die rasche Abheilung nicht beeinträchtigt. Diese mehrwertigen Alkohole bewirken eine Verminderung des Dampfdrucks, so daß die erfindungsgemäßen Mittel nicht austrocknen. Hierdurch wird ein Verkleben von Wunden bzw. Wundrändern vermieden, so daß dieses Mittel problemlos wieder entfernt werden kann. Darüberhinaus bewirken diese mehrwertigen Alkohole, daß die Gelzubereitung pastöser wird und somit leichter appliziert werden kann. Das Gel in den erfindungsgemäßen Formulierungen ist in üblicher Weise sterilisierbar, sein pH-Wert wird so eingestellt, daß er in dem therapeutisch besonders wünschenswerten Bereich zwischen 6,0 und 7,5 liegt.

Die nachfolgenden Ausführungsbeispiele dienen zur näheren Erläuterung des erfindungsgemäßen Mittels.

Beispiel 1

2,000 g Pfropf-Copolymerisat SGP 157 M der Firma Henkel Corporation, Minneapolis, werden mit methanolischer Ammoniaklösung gewaschen und mittels verdünnter Salzsäure auf einen pH-Wert von 6,8 eingestellt. Dann wird das Polymerisat mit 68,00 g demineralisiertem Wasser und 30,00 g Glycerin (DAB 8) verrührt, bis eine pastöse Masse entsteht. Diese wird im Druckautoklaven bei 125°C sterilisiert.

Beispiel 2

2,000 g Pfropf-Copolymerisat SGP 157 M der Fima Henkel Corporation, Minneapolis, werden mit methanolischer Ammoniaklösung gewaschen und mittels verdünnter Salzsäure auf einen pH-Wert von 6,8 eingestellt. Dann wird das Polymerisat mit 68,00 g demineralisiertem Wasser und 30,00 g Polyethylenglykol vom Molgewicht 600 verrührt, bis eine pastöse Masse entsteht. Diese wird im Druckautoklaven bei 125°C sterilisiert.

Beispiel 3

2,000 g Pfropf-Copolymerisat SGP 157 M der Firma Henkel Corporation, Minneapolis, werden mit methanolischer Ammonikalösung gewaschen und mittels verdünnter Salzsäure auf einen pH-Wert von 6,8 eingestellt. Dann wird das Polymerisat mit 78,00 g demineralisiertem Wasser und 20,00 g 1,2-Dihydroxypropan verrührt, bis eine pastöse Masse entsteht. Diese wird im Druckautoklaven bei 125°C sterilisiert.

**Patentansprüche**

1. Mittel zur Behandlung von Wunden, dadurch gekennzeichnet, daß es 1 bis 4 Gewichtsprozent eines Pfropf-Copolymerisats aus Stärke und hydrolysiertem Polyacrylnitril, dessen Carboxylgruppen-Wasserstoffe zu 5 bis 90% durch Aluminium substituiert sind, in einer 2 bis 50 gewichtsprozentigen wassrigen Di- oder Polyhydroxyalkan-Lösung, enthält.

2. Mittel zur Wundbehandlung gemäß Patentanspruch 1, dadurch gekennzeichnet, daß es als Dihydroxyalkan ein Glykol mit 2 bis 6 Kohlenstoffatomen enthält.

3. Mittel zur Behandlung von Wunden gemäß Patentanspruch 1, dadurch gekennzeichnet, daß es als Polyhydroxyalkan ein Polyol der allgemeinen Formel

$$C_nH_{(2n+2)}O_n$$

worin n eine Ziffer zwischen 3 und 6 bedeutet, enthält.

4. Mittel zur Behandlung von Wunden gemäß Patentanspruch 1, dadurch gekennzeichnet, daß es als Polyhydroxyalkan ein wasserlösliches Polyethylenglykol oder Polypropylenglykol enthält.

5. Mittel zur Behandlung von Wunden gemäß Patentanspruch 1 bis 4, dadurch gekennzeichnet, daß es zusätzlich ein oder mehrere Wirkstoffe in therapeutisch wirksamen Mengen enthält.

6. Mittel zur Behandlung von Wunden, gemäß Patentanspruch 5, dadurch gekennzeichnet, daß der Wirkstoff ein Bakteriostatikum ist.

7. Mittel zur Behandlung von Wunden gemäß Patentanspruch 5, dadurch gekennzeichnet, daß es als Wirkstoff ein Antimykotikum enthält.

8. Mittel zur Behandlung von Wunden gemäß Patentanspruch 4, dadurch gekennzeichnet, daß der Wirkstoff ein Lokalanaesthetikum ist.

**Revendications**

1. Agent pour le traitement des plaies caractérisé en ce qu'il contient 1 à 4 pour cent en poids d'un copolymère gréffé d'un amidon et d'un polyacrylonitrile hydrolisé dont 5 à 90% des hydrogènes présents dans les groupes carboxyliques ont été substitués par de l'aluminium, dans une solution aqueuse contenant de 2 à 50 pour cent en poids d'un di- ou polyhydroxyalcane.

2. Agent pour le traitement des plaies selon la revendication 1 caractérisé en ce que le dihydroxyalcane est un glycol ayant de 2 à 6 atomes de carbone.

3. Agent pour le traitement des plaies selon la revendication 1 caractérisé en ce que le polyhydroxyalcane est un polyol de formule générale:

$$C_nH_{(2n+2)}O_n$$

dans lequel n est un nombre compris entre 3 et 6.

4. Agent pour le traitement des plaies selon la revendication 1 caractèrisé, en ce que le polyhydroxyalcane est un polyéthylèneglycol ou un polypropylèneglycol soluble dans l'eau.

5. Agent pour le traitement des plaies selon les revendications 1 à 4 caractérisé en ce qu'il contient un ou plusieurs principes actifs en quantité thérapeutiquement active.

6. Agent pour le traitement des plaies selon les revendications 5 caractérisé en ce que l'agent actif est un bactériostatique.

7. Agent pour le traitement des plaies selon la revendication 5 caractérisé en ce que l'agent actif est un antimicotique.

8. Agent pour le traitement des plaies selon la revendication 4 caractérisé en ce que l'agent actif est un anesthésique local.

**Claims**

1. Agent for the treatment of wounds comprising 1 to 4 weight percent of a graft copolymer made of starch and hydrolysed polyacrylnitrile, 5 to 90% of the carboxylic hydrogen thereof being substituted by aluminium, in a 2 to 50 weight percent aqueous solution of a di- or polyhydroxyalkane.

2. Agent for the treatment of wounds according to Claim 1 characterized in that it comprises a glycol having 2 to 6 carbon atoms as a dihydroxyalkane.

3. Agent for the treatment of wounds according to Claim 1 characterized in that it comprises as a polyhydroxyalkane a polyol of the general formula

$$C_nH_{(2n+2)}O_n$$

wherein n stands for a numeral between 3 and 6.

4. Agent for the treatment of wounds according to Claim 1 characterized in that it comprises a water-soluble polyethyleneglycol or polypropyleneglycol as a polyhydroxyalkane.

5. Agent for the treatment of wounds according to Claims 1 to 4 characterized in that it additionally comprises one or more active agents in a therapeutically active amount.

6. Agent for the treatment of wounds according to Claim 5 characterized in that the active agent is a bacteriostat.

7. Agent for the treatment of wounds according to Claim 5 characterized in that it comprises an antimycotic as an active agent.

8. Agent for the treatment of wounds according to Claim 4 characterized in that the active agent is a local anaesthetic.